# EUROPÄISCHE PATENTSCHRIFT

(11) **EP 0 438 691 B1**
(45) Veröffentlichungstag und Bekanntmachung des Hinweises auf die Patenterteilung: **16.02.1994**
(21) Anmeldenummer: 90123776.8
(22) Anmeldetag: 11.12.1990
(51) Int. Cl.: C07D 213/61

(54) **Verfahren zur Herstellung von substituierten 2-Chlorpyridinen**
Process for the preparation of substituted 2-chloropyridines
Procédé pour la préparation de chloro-2 pyridines substituées

(30) Priorität: 18.01.1990 DE 4001248; 23.06.1990 DE 4020055
(43) Veröffentlichungstag der Anmeldung: 31.07.1991
(73) Patentinhaber: BAYER AG, 51368 Leverkusen (DE)
(72) Erfinder: Kaufmann, Dieter, Dr., W-5060 Bergisch-Gladbach 2 (DE); Jelich, Klaus, Dr., W-5600 Wuppertal 1 (DE); Braden, Rudolf, Dr., W-5068 Odenthal (DE); Rosen, Winfried, Dr., W-5600 Wuppertal 1 (DE)

(56) Entgegenhaltungen:
- DE-B- 1 200 304
- US-A- 4 628 096
- CHEMICAL ABSTRACTS, Band 58, Nr. 1, 7.Januar 1963, Columbus, Ohio, USA VOZZA J.F. "Reactions of 2- picoline-1-oxide with hali- des" Spalte 500,Zusammenfassung- Nr. 500b

## Beschreibung

Die vorliegende Erfindung betrifft ein neues Verfahren zur Herstellung von substituierten 2-Chlorpyridinen.

Es ist bekannt, daß man 2-Chlor-5-methyl-pyridin neben 2-Chlor-3-methyl-pyridin, 4-Chlor-3-methyl-pyridin und 3-Chlor-5-methyl-pyridin erhält, wenn man 3-Methyl-pyridin-1-oxid mit Phosphorylchlorid umsetzt (vgl. Weissberger, Chemistry of Heterocyclic Compounds, Pyridine and its Derivatives, Vol. 14, Supplement, Part 2, S. 112, Verlag John Wiley & Sons, New York, 1974). Hauptprodukt dieser Umsetzung ist 4-Chlor-3-methyl-pyridin; der Anteil an 2-Chlor-5-methyl-pyridin liegt im allgemeinen unter 25 %.

Die Umsetzung von 3-Methyl-pyridin-1-oxid mit Phosphorylchlorid in Gegenwart einer basischen organischen Stickstoffverbindung und in Gegenwart eines Verdünnungsmittels ist bekannt (Deutsche Offenlegungsschrift 3 800 179) und die Umsetzung substituierter Pyridin-1-oxide mit Phosphorsäureesterchloriden oder Phosphorsäureamidchloriden in Gegenwart einer basischen organischen Stickstoffverbindung und in Gegenwart eines Verdünnungsmittels ist Gegenstand einer älteren Patentanmeldung (Deutsche Patentanmeldung P 38 39 332 vom 22.11.1988).

Es wurde nun ein neues Verfahren zur Herstellung von substituierten 2-Chlorpyridin-Derivaten der Formel (I) 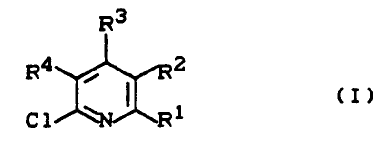
in welcher
- R¹: für Wasserstoff, Fluor, Chlor, Cyano, Carbalkoxy (C₁-C₄) oder 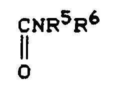 steht,
worin R⁵ und R⁶ gleich oder verschieden sind und für Wasserstoff oder Alkyl (C₁-C₄) stehen,
- R²: für Wasserstoff, Fluor, Chlor, Alkyl(C₁-C₄), Halogenalkyl-(C₁-C₄), Cyanalkyl(C₁-C₄), Alkoxy(C₁-C₄)-alkyl(C₁-C₄), Dialkylamino(C₁-C₄)alkyl(C₁-C₄), Acyl(C₁-C₄)-acetal; Carbalkoxy-(C₁-C₄) oder 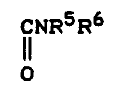 steht,
worin R⁵ und R⁶ gleich oder verschieden sind und für Wasserstoff oder Alkyl (C₁-C₄) stehen oder worin R¹ und R2 zusammen für die bivalente Gruppe -CH=CH-CH=CH- stehen,
- R³: für Wasserstoff, Chlor, Carbalkoxy(C₁-C₄) oder 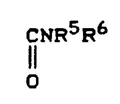 steht,
worin R⁵ und R⁶ gleich oder verschieden sind und für Alkyl (C₁-C₄) stehen,
- R⁴: für Wasserstoff, Fluor, Chlor oder Cyano steht, oder worin R³ und R⁴ zusammen für die bivalente Gruppe -CH=CH-CH=CH- stehen,

gefunden.

Das neue Verfahren ist dadurch gekennzeichnet, daß man Pyridin-1-oxide der Formel II 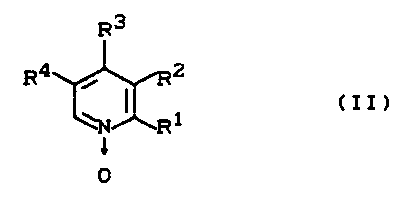
in welcher
R¹, R², R³ und R⁴ die oben angegebene Bedeutung haben, mit einem aromatischen Carbonsäurechlorid in Gegenwart eines inerten organischen Lösungsmittels und in Gegenwart eines Säureakzeptors bei Temperaturen zwischen -20° C und 200° C umsetzt, darauf gegebenenfalls bei Temperaturen zwischen 70° C und 160° C mit Phosgen oder Thionylchlorid umsetzt und das erhaltene Produkt gegebenenfalls weiter auftrennt.

Gegebenenfalls wird bei der Umsetzung mit Phosgen oder Thionylchlorid in Gegenwart eines Aktivators gearbeitet.

Überraschenderweise gelingt es mit Hilfe des erfindungsgemäßen Verfahrens, auf einfache Weise und mit geringem Aufwand unter Erzielung überraschend hoher Ausbeuten, substituierte Pyridin-1-oxide der Formel (II) durch Umsetzung mit Benzoylchloriden bzw. Phthaloylchloriden in Gegenwart eines Säureakzeptors gegebenenfalls unter Nachbehandlung mit Phosgen oder Thionylchlorid in die entsprechenden substituierten 2-Chlorpyridine der Formel (I) zu überführen.

Vorteile des erfindungsgemäßen Verfahrens liegen neben der guten Ausbeute am gewünschten Produkt auch darin, daß der Anteil an isomeren Nebenprodukten wesentlich geringer als bei den bisher bekannten Synthesemethoden ist. Ein weiterer Vorteil besteht darin, daß es sich bei den erfindungsgemäß eingesetzten Chlorierungsmitteln um gängige technische Produkte handelt, und diese nach der Chlorierung der Pyridin-N-oxide sogar wieder rückgeführt werden können. Weiterhin kann man, falls erwünscht, aus dem Reaktionsprodukt die reine Verbindung (I) leicht durch übliche Methoden, z.B. durch destillative Trennung oder durch andere übliche Trennmethoden herstellen.

Das erfindungsgemäße Verfahren stellt somit eine wertvolle Bereicherung der Technik dar.

Setzt man 3-Methyl-pyridin-1-oxid mit ortho-Phthaloylchlorid in Gegenwart der Base Triethylamin in Methylenchlorid um, so kann der Reaktionsablauf beim erfindungsgemäßen Verfahren durch das nachstehende Formelschema skizziert werden.

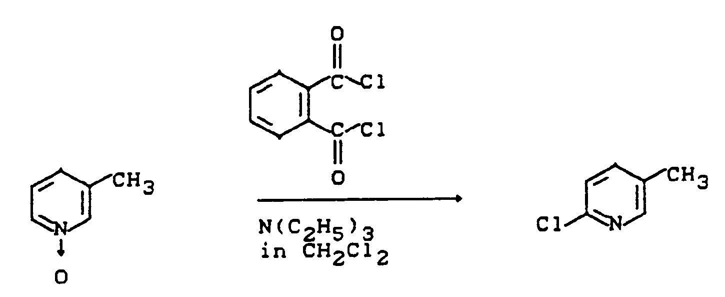

Als nach dem erfindungsgemäßen Verfahren bevorzugt herstellbare Verbindungen der allgemeinen Formel (I) seien genannt:
2,3-Dichlorpyridin
2,4-Dichlorpyridin
2,6-Dichlorpyridin
2-Chlor-3-cyan-pyridin
2-Chlor-6-cyan-pyridin
6-Chlor-pyridin-2-carbonsäuremethylester
6-Chlor-pyridin-2-carbonsäureethylester
6-Chlor-pyridin-2-carbonsäuredimethylamid
6-Chlor-pyridin-2-carbonsäurediethylamid
6-Chlornicotinsäurediethylamid
2-Chlor-pyridin-4-carbonsäuremethylester
2-Chlor-pyridin-4-carbonsäureethylester
2-Chlor-pyridin-4-carbonsäuredimethylamid
2-Chlor-pyridin-4-carbonsäurediethylamid
2-Chlor-5-diisopropylaminomethyl-pyridin
2-Chlor-5-diisobutylaminomethyl-pyridin
2-Chlor-5-methyl-pyridin
2,6-Dichlor-3-methyl-pyridin
2,6-Dichlor-3-fluor-5-methyl-pyridin
2-Chlor-3,6-difluor-5-methyl-pyridin
2-Chlor-5-ethyl-pyridin
2-Chlor-5-chlormethyl-pyridin
2-Chlor-5-acetyl-pyridin-ethylenacetal
2,3-Dichlor-5-acetyl-pyridin-ethylenacetal
2-Chlor-5-cyanmethyl-pyridin
2-Chlor-5-methoxymethyl-pyridin
2-Chlor-5-ethoxymethyl-pyridin
6-Chlor-nicotinsäuremethylester
6-Chlor-nicotinsäureethylester
6-chlor-nicotinsäuredimethylamid
2-Chlorchinolin
1-Chlorisochinolin

Besonders bevorzugt wird folgende Verbindung der Formel (I) hergestellt:
2-Chlor-5-methyl-pyridin.

Die Ausgangsprodukte Pyridin-1-oxide der Formel (II) sowie Benzoylchloride bzw, Phthaloylchloride sind bekannte Verbindungen der organischen Chemie oder lassen sich durch bekannte Verfahren herstellen. Siehe z.B.:

Weissberger, Chemistry of Heterocyclic Compounds, Pyridine and its Derivatives, Vol. 14, Supplement, Part 2, S. 112, Verlag John Wiley & Sons, New York, 1974.

Methoden der Organischen Chemie (Houben-Weyl, Müller), Band VIII, S. 463 ff., Georg Thieme Verlag, Stuttgart, 1952.

Die als Ausgangsstoffe für das erfindungsgemäße Verfahren eingesetzten substituierten Pyridin-1-oxide sind durch die Formel (II) allgemein definiert.

In der Formel (I) steht
- R¹: vorzugsweise für Wasserstoff, Fluor, Chlor, Cyano, COOCH₃, COOC₂H₅, CON(CH₃)₂, CON(C₂H₅)₂,
- R²: vorzugsweise für Wasserstoff, Fluor, Chlor, CH₃, C₂H₅, CH₂Cl, CH₂CN, CH₂-OCH₃, CH₂-OC₂H₅, CH₂-N(i-C₃H₇)₂, CH₂-N(i-C₄H₉)₂, 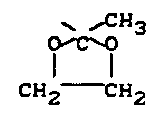 COOCH₃, COOC₂H₅, CON(CH₃)₂, CON(C₂H₅)₂,
- R³: vorzugsweise für Wasserstoff, Chlor, COOCH₃, COOC₂H₅, CON(CH₃)₂, CON(C₂H₅)₂

oder
- R¹ und R²: stehen jeweils zusammen für die bivalente Gruppe -CH=CH-CH=CH-,
- R⁴: steht vorzugsweise für Wasserstoff, Fluor, Chlor, Cyan oder R³ und R⁴ jeweils zusammen für die bivalente Gruppe -CH=CH-CH=CH-.

Insbesondere wird 3-Methyl-pyridin-1-oxid als Ausgangsstoff der Formel (II) eingesetzt.

Die beim erf indungsgemäßen Verfahren eingesetzten aromatischen Carbonsäurechloride entstammen vorzugsweise folgenden Verbindungen bzw. Verbindungsklassen:
Benzoylchlorid
Monochlorbenzoylchloride
Dichlorbenzoylchloride
Monomethylbenzoylchloride
Monochlormethylbenzoylchloride
Dimethylbenzoylchloride
Monomethoxybenzoylchloride
Mononitrobenzoylchloride
Phthaloylchloride
Dichlorphthalid
Monochlorphthaloylchloride
Dichlorphthaloylchloride
Mononitrophthaloylchloride
Naphthalincarbonsäurechloride.

Besonders bevorzugt werden Benzoylchlorid und ortho-, meta- und para-Phthaloylchlorid eingesetzt.

Das erfindungsgemäße Verfahren wird unter Verwendung von inerten organischen Lösungsmitteln durchgeführt. Es kommen dabei praktisch alle inerten organischen Lösungsmittel in Frage. Hierzu gehören vorzugsweise aliphatische oder aromatische Kohlenwasserstoffe wie Pentan, Hexan, Heptan, Octan, Cyclohexan, Methylcyclohexan, Petrolether, Benzin, Ligroin, Benzol, Toluol, Xylol und Tetralin, halogenierte Kohlenwasserstoffe wie Methylenchlorid, Ethylenchlorid, Trichlorethylen, Chloroform, Tetrachlorkohlenstoff, 1,1,2-Trichlorethan, 1,2-Dichlorpropan, 1,2,3-Trichlorpropan, Chlorbenzol und Dichlorbenzol, Ether wie Diethylether, Dipropylether, Diisopropylether, Dibutylether, Methyl-tert-butylether, Methyl-tert.-amylether, Glykoldimethylether, Diglykoldimethylether, Tetrahydrofuran und Dioxan und Anisol, Ketone wie Aceton, Methylethylketon, Diethylketon, Methyl-isopropylketon, Methyl-isobutylketon und Methyltert.-butylketon, Ester wie Essigsäuremethylester, Essigsäureethylester, Essigsäurepropylester, Essigsäurebutylester, Essigsäureamylester, Phthalsäuredimethylester und Phthalsäurediethylester, Nitrile wie Acetonitril und Propionitril, Amide wie Dimethylformamid, Dimethylacetamid und N-Methylpyrrolidon sowie Dimethylsulfoxid und Tetramethylensulfon.

Methylenchlorid, Ethylenchlorid, Chloroform, 1,1,2-Trichlorethan, 1,2-Dichlorpropan, 1,2,3-Trichlorpropan und Chlorbenzole werden als organische Lösungsmittel besonders bevorzugt.

Als Säureakzeptoren werden beim erfindungsgemäßen Verfahren vorzugsweise basische organische Stickstoffverbindungen eingesetzt: so Trialkylamine wie z.B. Trimethylamin, Triethylamin, Tripropylamin, Tributylamin und Diazabicyclooctan, Dialkylcycloalkylamine wie z.B. Dimethyl-cyclopentylamin, Diethyl-cyclopentylamin, Dimethyl-cyclohexylamin und Diethyl-cyclohexylamin, Dialkyl-aralkylamin wie z.B. Dimethylbenzylamin und Diethylbenzylamin.

Triethylamin wird als basische organische Stickstoffverbindung besonders bevorzugt.

Beim erfindungsgemäßen Verfahren arbeitet man in einem Temperaturbereich zwischen -20° C und 200° C, vorzugsweise bei Temperaturen zwischen 0° C und 160° C. Das erfindungsgemäße Verfahren wird im allgemeinen unter Normaldruck durchgeführt. Es ist jedoch auch möglich, unter erhöhtem oder vermindertem Druck zwischen 0,1 und 10 bar zu arbeiten. Zur Durchführung des erfindungsgemäßen Verfahrens setzt man auf 1 Mol substituiertes Pyridin-1-oxid der Formel (II) im allgemeinen zwischen 1 und 10 Mol, vorzugsweise zwischen 1 und 2 Mol, aromatisches Carbonsäurechlorid und ebenfalls zwischen 1 und 10 Mol, vorzugsweise zwischen 1 und 2 Mol des Säureakzeptors, vorzugsweise der basischen organischen Stickstoffverbindung, ein. Der Einsatz von angenähert je 1,5 Mol aromatischem Carbonsäurechlorid und Stickstoffverbindung je Mol substituiertem Pyridin-1-oxid wird besonders bevorzugt. Beim Arbeiten in Chlorbenzolen ist es vorteilhaft anschließend zur Erzielung einer höheren Ausbeute noch mit Phosgen oder Thionylchlorid umzusetzen. Zur Durchführung dieses erfindungsgemäßen Verfahrensschrittes setzt man auf 1 Mol substituiertes Pyridin-1-oxid der Formel (II) im allgemeinen zwischen 1 und 10 Mol, vorzugsweise zwischen 2 und 5 Mol, Phosgen oder Thionylchlorid ein; besonders bevorzugt ist der Einsatz von 2 bis 4 Mol Phosgen. Das Phosgen und das Thionylchlorid kann gegebenenfalls durch Zusatz eines katalytisch wirksamen Aktivators aktiviert werden. Bevorzugt werden hierzu Verbindungen aus der Klasse der Formamide und besonders bevorzugt wird das N,N-Dibutylformamid oder das N-Methylformanilid oder Distearylformamid zugesetzt.

Zur bevorzugten Durchführung des erfindungsgemäßen Verfahrens tropft man zu einer Lösung des substituierten Pyridin-1-oxids der Formel (II) und der basischen organischen Stickstoffverbindung das aromatische Carbonsäurechlorid, und das komplette Reaktionsgemisch wird mehrere Stunden bei der jeweiligen Temperatur (vorzugsweise im Bereich von 40 bis 160° C) gerührt. Beim Arbeiten in Chlorbenzolen setzt man zur bevorzugten Durchführung des erfindungsgemäßen Verfahrens anschließend ein Formamid zu und leitet mehrere Stunden bei der jeweiligen Temperatur (vorzugsweise im Bereich von 70 bis 150° C) Phosgen ein bzw. tropft Thionylchlorid zu. Beim Arbeiten in Chlorbenzol ist eine Temperatur von 120 bis 140° C besonders bevorzugt.

Die Aufarbeitung kann auf übliche Weise durchgeführt werden. Vorzugsweise wird zuerst das aus der basischen organischen Stickstoffverbindung gebildete Hydrochlorid abfiltriert; es kann nach alkalischer Spaltung wieder rückgeführt werden. Dann wird vorzugsweise das organische Lösungsmittel - beispielsweise durch Destillation - entfernt, der Sumpf mit einer wäßrigen Alkali- oder Erdalkalimetallhydroxidlösung wie z.B. Natronlauge auf pH 6 eingestellt und das Reaktionsprodukt daraus weitgehend durch Wasserdampfdestillation entfernt. Der organische Anteil des Wasserdampfdestillats enthält im wesentlichen das Produkt der Formel (I).

Die Reindarstellung der Verbindung der Formel (I) aus dem organischen Anteil des Wasserdampfdestillats kann nach üblichen Methoden, beispielsweise durch eine Feindestillation an einer Füllkörperkolonne erfolgen. Die Gesamtausbeute bei der Herstellung von 2-Chlor-5-methyl-pyridin beträgt ausgehend von 3-Methyl-pyridin-1-oxid 60 bis 85 % der Theorie.

Das nach dem erfindungsgemäßen Verfahren herstellbare 2-Chlor-5-methyl-pyridin ist als Zwischenprodukt für Pharmazeutika bekannt (vgl. DE-A 28 12 585).

Weiterhin kann 2-Chlor-5-methyl-pyridin vorteilhaft als Zwischenprodukt für die Herstellung von insektiziden Nitromethylen-Derivaten eingesetzt werden (vgl. EP-A 163 855).

### Herstellungsbeispiel 1

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml Methylenchlorid werden unter Stickstoff 76,1 g (0,375 Mol) o-Phthaloylchlorid in 45 min so getropft, daß leichter Rückfluß eintritt. Anschließend wird noch 2 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation unterworfen, wobei durch kontinuierliche Zugabe von 45 %iger Natronlauge der pH-Wert 6 eingehalten wird. Das Destillat wird dreimal mit je 100 ml Methylenchlorid extrahiert, die Extrakte werden fraktioniert destilliert.
- Ausbeute:: 27,1 g (85 %) eines Gemisches aus 84 % 2-Chlor-5-methylpyridin und 16 % 2-Chlor-3-methylpyridin.

Die Struktur wird durch ¹H-NMR-Spektren gesichert.

¹H-NMR (CDCl₃, 200 MHz): δ = 8,16 (6-H, ddq), 7,43 (4-H, ddq), 7,16 (3-H, br. d), 2,26 ppm (CH₃, br. s).

Das reine 2-Chlor-5-methyl-pyridin kann durch fraktionierte Destillation abgetrennt werden.

### Herstellungsbeispiel 2

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml Chlorbenzol werden unter Stickstoff 76,1 g (0,375 Mol) o-Phthaloylchlorid in 30 min getropft. Anschließend wird 2,5 Stunden unter Rückfluß erhitzt, dann abgesaugt und der Filterkuchen mit 50 ml Chlorbenzol gewaschen. Nach Zugabe von 2 ml N,N-Dibutylformamid wird bei 120°C in 2 Stunden 98,9 9 (1 Mol) Phosgen eingeleitet. Man läßt abkühlen, verrührt 30 min mit 200 ml konzentrierter Salzsäure, neutralisiert mit 45 %iger Natronlauge, extrahiert die wäßrige Phase dreimal mit je 100 ml Toluol und fraktioniert die Toluolphase.
- Ausbeute:: 22,3 g (70 %) eines Gemisches aus 85 % 2-Chlor-5-methylpyridin und 15 % 2-Chlor-3-methylpyridin.

Das reine 2-Chlor-5-methyl-pyridin kann durch fraktionierte Destillation abgetrennt werden.

### Herstellungsbeispiel 3

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml Methylenchlorid werden unter Stickstoff 52,7 g (0,375 Mol) Benzoylchlorid in 45 min getropft. Anschließend wird noch 3 Stunden unter Rückfluß erhitzt, dann filtriert, der Filterkuchen mit 50 ml Methylenchlorid gewaschen und das Lösungsmittel abdestilliert. Der Sumpf wird einer Wasserdampfdestillation unterworfen, wobei durch kontinuierliche Zugabe von 45 %iger Natronlauge der pH-Wert 6 eingehalten wird. Das Destillat wird dreimal mit je 100 ml Methylenchlorid extrahiert, die Extrakte werden fraktioniert destilliert.
- Ausbeute:: 21,7 g (68 %) eines Gemisches aus 87 % 2-Chlor-5-methylpyridin und 13 %. 2-Chlor-3-methylpyridin.

Das reine 2-Chlor-5-methyl-pyridin kann durch fraktionierte Destillation abgetrennt werden.

### Herstellungsbeispiel 4

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml Chlorbenzol werden unter Stickstoff 52,7 g (0,375 Mol) Benzoylchlorid in 30 min getropft. Anschließend wird 3 Stunden unter Rückfluß erhitzt, abgesaugt und der Filterkuchen mit 50 ml Chlorbenzol gewaschen. Nach Zugabe von 2 ml N,N-Dibutylformamid wird bei 120°C in 2 Stunden 98,9 g (1 Mol) Phosgen eingeleitet. Man läßt abkühlen, verrührt 30 min mit 200 ml konz. Salzsäure, neutraliciert mit 45 proz. Natronlauge, extrahiert die wäßrige Phase dreimal mit je 100 ml Toluol und fraktioniert die Toluolphase.
- Ausbeute:: 19,1 g (60 %) eines Gemisches aus 86 % 2-Chlor-5-methylpyridin und 14 % 2-Chlor-3-methylpyridin.

Das reine 2-Chlor-5-methyl-pyridin kann durch fraktionierte Destillation abgetrennt werden.

### Herstellungsbeispiel 5

### 2-Chlor-5-methyl-pyridin

Zu einer Lösung von 27,3g (0,25 Mol) 3-Methylpyridin-1-oxid und 37,9 g (0,375 Mol) Triethylamin in 250 ml 1,2-Dichorpropan werden unter Stickstoff 76,1 g (0,375 Mol) o-Phthaloylchlorid in 45 min getropft. Anschließend wird noch 4 Stunden unter Rückfluß erhitzt, dann abgesaugt, der Filterkuchen mit 50 ml 1,2-Dichlorpropan gewaschen und das Lösungsmittel abdestilliert. Der Sumpf wird einer Wasserdampfdestillation unterworfen, wobei durch kontinuierliche Zugabe von 45 proz. Natronlauge der pH-Wert 6 eingehalten wird. Das Destillat wird dreimal mit je 100 ml Methylenchlorid extrahiert, die Extrakte werden fraktioniert destilliert.
- Ausbeute:: 22,3 g (70 %) eines Gemisches aus 84 % 2-Chlor-5-methylpyridin und 16 % 2-Chlor-3-methylpyridin.

Das reine 2-Chlor-5-methyl-pyridin kann durch fraktionierte Destillation abgetrennt werden.

### Herstellungsbeispiel 6

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml 1,2,3-Trichlorpropan werden unter Stickstoff 76,1 g (0,375 Mol) o-Phthaloylchlorid in 45 min getropft. Anschließend wird noch 12 Stunden auf 70°C erhitzt, dann abgesaugt und der Filterkuchen mit 50 ml 1,2,3-Trichlorpropan gewaschen. Die flüssige Phase wird mit 100 ml konz. Salzsäure verrührt, die wäßrige Phase abgetrennt, mit Natronlauge neutralisiert und dreimal mit je 50 ml Toluol extrahiert; die Extrakte werden fraktioniert destilliert.
- Ausbeute:: 25,4 g (80 %) eines Gemisches aus 84 % 2-Chlor-5-methylpyridin und 16 % 2-Chlor-3-methylpyridin.

Das reine 2-Chlor-5-methyl-pyridin kann durch fraktionierte Destillation abgetrennt werden.

### Herstellungsbeispiel 7

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml Methylenchlorid werden unter Stickstoff 65,6 g (0,375 Mol) 4-Chlorbenzoylchlorid in 35 min getropft. Anschließend wird noch 5 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation unterworfen, wobei durch kontinuierliche Zugabe von 45 proz. Natronlauge der pH-Wert 6 eingehalten wird. Das Destillat wird dreimal mit je 100 ml Methylenchlorid extrahiert, die Extrakte werden fraktioniert destilliert.
- Ausbeute:: 21,6 g (68 %) eines Gemisches aus 89 % 2-Chlor-5-methylpyridin und 11 % 2-Chlor-3-methylpyridin.

Das reine 2-Chlor-5-methyl-pyridin kann durch fraktionierte Destillation abgetrennt werden.

### Herstellungsbeispiel 8

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml Methylenchlorid werden unter Stickstoff 76,9 g (0,375 Mol) 2-Chlormethyl-benzoylchlorid in 30 min. getropft. Anschließend wird noch 5 Stunden unter Rückfluß erhitzt, dann abgesaugt, der Filterkuchen mit 100 ml Methylenchlorid gewaschen und das Lösungsmittel abdestilliert. Der Sumpf wird einer Wasserdampfdestillation unterworfen, wobei durch kontinuierliche Zugabe von 45 proz. Natronlauge der pH-Wert 6 eingehalten wird. Das Destillat wird dreimal mit je 100 ml Methylenchlorid extrahiert, die Extrakte werdenfraktioniert destilliert.
- Ausbeute:: 24,8 g (78 %) eines Gemisches aus 87 % 2-Chlor-5-methylpyridin und 13 % 2-Chlor-3-methylpyridin.

Das reine 2-Chlor-5-methylpyridin kann durch fraktionierte Destillation abgetrennt werden.

### Herstellungsbeispiel 9

### 2-Chlor-5-methyl-pyridin

Zu einer Lösung von 27,3 g (0,25 Mol) 3-Methylpyridin-1-oxid und 37,9 g (0,375 Mol) Triethylamin in 250 ml Methylenchlorid werden unter Stickstoff 76,1 g (0,375 Mol) m-Phthaloylchlorid in 30 min. getropft. Anschließend wird noch 5 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation unterworfen, wobei durch kontinuierliche Zugabe von 45 proz. Natronlauge der pH-Wert 6 eingehalten wird. Das Destillat wird dreimal mit je 100 ml Methylenchlorid extrahiert, die Extrkte werden fraktioniert destilliert.
- Ausbeute:: 23,9 g (75 %) eines Gemicches aus 89 % 2-Chlor-5-methylpyridin und 11 % 2-Chlor-3-methylpyridin.

Das reine 2-Chlor-5-methyl-pyridin kann durch fraktionierte Destillation abgetrennt werden.

### Herstellungsbeispiel 10

### 2-Chlor-5-butyl-pyridin

Zu einer Lösung von 37,9 g (0,25 Mol) 3-Butylpyridin-1-oxid und 37,9 g (0,375 Mol) Triethylamin in 250 ml Methylenchlorid werden unter Stickstoff 76,1 g (0,375 Mol) o-Phthaloylchlorid in 30 min getropft. Anschließend wird noch 5 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation unterworfen, wobei durch kontinuierliche Zugabe von 45 proz. Natronlauge der pH-Wert 6 eingehalten wird. Das Destillat wird dreimal mit je 100 ml Toluol extrahiert, die Extrakte werden fraktioniert destilliert.
- Ausbeute:: 32,2 g (76 %) eines Gemisches aus 95 % 2-Chlor-5-butylpyridin und 5 % 2-Chlor-3-butylpyridin.

### Herstellungsbeispiel 11

### 2,6-Dichlor-3-methyl-pyridin

Zu einer Lösung von 35,9 g (0,25 Mol) 2-Chlor-5-methyl-pyridin-1-oxid und 50,6 g (0,50 Mol) Triethylamin in 200 ml Chlorbenzol werden unter Stickstoff 101,5 g (0,50 Mol) o-Phthaloylchlorid in 50 ml Chlorbenzol in 30 min. getropft. Anschließend wird noch 12 Stunden unter Rückfluß erhitzt, dann abgesaugt, der Filterkuchen mit 50 ml Methylenchlorid gewaschen und das Lösungsmittel abdestilliert. Der Sumpf wird einer Wasserdampfdestillation unterworfen, wobei durch kontinuierliche Zugabe von 45 proz. Natronlauge der pH-Wert 6 eingehalten wird. Das Destillat wird dreimal mit je 100 ml Methylenchlorid extrahiert, das Lösungsmittel abdestilliert und der Rückstand aus Hexan umkristallisiert.
- Ausbeute:: 32,2 g (68 %) 2,6-Dichlor-3-methylpyridin.

## Patentansprüche

1. Verfahren zur Herstellung von substituierten 2-Chlorpyridin-Derivaten der Formel (I) 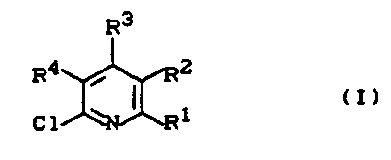 in welcher
R¹ für Wasserstoff, Fluor, Chlor, Cyano, Carbalkoxy (C₁-C₄) oder 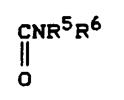 steht,
worin R⁵ und R⁶ gleich oder verschieden sind und für Alkyl (C₁-C₄) stehen,
R² für Wasserstoff, Fluor, Chlor, Alkyl(C₁-C₄), Halogenalkyl(C₁-C₄), Cyanalkyl(C₁-C₄), Alkoxy(C₁-C₄)alkyl(C₁-C₄), Dialkylamino(C₁-C₄)alkyl(C₁-C₄), Acyl(C₁-C₄)-acetal, Carbalkoxy-(C₁-C₄) oder 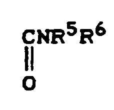 steht,
worin R⁵ und R⁶ gleich oder verschieden sind und für Alkyl (C₁-C₄) stehen
oder worin R¹ und R² zusammen für die bivalente Gruppe -CH=CH-CH=CH- stehen,
R³ für Wasserstoff, Chlor, Carbalkoxy(C₁-C₄) oder 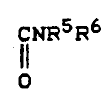 steht,
worin R⁵ und R⁶ gleich oder verschieden sind und für Alkyl (C₁-C₄) stehen,
R⁴ für Wasserstoff, Fluor, Chlor oder Cyano steht,
oder worin R³ und R⁴ zusammen für die bivalente Gruppe -CH=CH-CH=CH- stehen,
dadurch gekennzeichnet, daß man Pyridin-1-oxide der Formel (II) 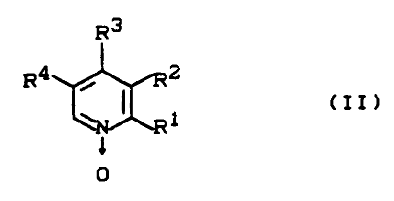 in welcher
R¹, R², R³ und R⁴ die oben angegebene Bedeutung haben, mit einem aromatischen Carbonsäurechlorid in Gegenwart eines inerten organischen Lösungsmittels und in Gegenwart eines Säureakzeptors bei Temperaturen zwischen -20°C und 200°C umsetzt, darauf gegebenenfalls bei Temperaturen zwischen 70°C und 160°C mit Phosgen oder Thionylchlorid, gegebenenfalls in Gegenwart eines Aktivators, umsetzt und das erhaltene Produkt gegebenenfalls weiter auftrennt.

2. Verfahren gemäß Anspruch 1, dadurch gekennzeichnet, daß bei der Umsetzung als aromatische Carbonsäurechloride Benzoylchlorid, Monochlorbenzoylchloride, Dichlorbenzoylchloride, Monomethylbenzoylchloride, Monochlormethylbenzoylchloride, Dimethylbenzoylchloride, Monomethoxybenzoylchloride, Mononitrobenzoylchloride, Phthaloylchloride, Dichlorphthalid, Monochlorphthaloylchloride, Dichlorphthaloylchloride, Mononitrophthaloylchloride, Naphthalincarbonsäurechloride eingesetzt werden.

3. Verfahren nach Anspruch 1 und 2, dadurch gekennzeichnet, daß man als Säureakzeptor eine basische organische Stickstoffverbindung der Reihe Trialkylamine, Dialkyl-cycloalkylamine und Dialkylaralkylamine einsetzt.

4. Verfahren gemäß Anspruch 1 bis 3, dadurch gekennzeichnt, daß als Säureakzeptor Triethylamin eingesetzt wird.

5. Verfahren gemäß Anspruch 1 bis 4, dadurch gekennzeichnet, daß als Aktivator eine Verbindung aus der Reihe der Formamide eingesetzt wird.

6. Verfahren gemäß Anspruch 1 bis 5, dadurch gekennzeichnet, daß als inerte organische Lösungsmittel Methylenchlorid, Ethylenchlorid, 1,1,2-Trichlorethan, 1,2-Dichlorpropan, 1,2,3-Trichlorpropan, Chloroform oder Chlorbenzol eingesetzt werden.

7. Verfahren gemäß Anspruch 1 bis 6, dadurch gekennzeichnet, daß die Umsetzung mit dem aromatischen Säurechlorid bei Temperaturen zwischen 0 und 160°C und gegebenenfalls anschließend mit Phosgen oder Thionylchlorid bei Temperaturen zwischen 70 und 160°C durchgeführt wird.

8. Verfahren gemäß Anspruch 1 bis 7, dadurch gekennzeichnet, daß man zu einer Lösung von 1 Mol des substituierten Pyridin-1-oxids der Formel (II) und von 1 bis 10 Mol eines Amins 1 bis 10 Mol eines aromatischen Carbonsäurechlorids tropft, gegebenenfalls anschließend ein Formamid zugibt und 2 bis 5 Mol Phosgen einleitet oder Thionylchlorid zutropft und das Reaktionsgemisch mehrere Stunden gerührt wird.

9. Verfahren gemäß Anspruch 1 bis 8, dadurch gekennzeichnet, daß man das bei der Umsetzung entstehende Reaktionsgemisch einer Wasserdampfdestillation unterwirft.

10. Verfahren gemäß Anspruch 1 bis 9, dadurch gekennzeichnet, daß man zur Herstellung von 2-Chlor-5-methylpyridin 3-Methyl-pyridin-1-oxid mit einem Benzoylchlorid oder Phthaloylchlorid unter Zugabe von Triethylamin als Säureakzeptor in Methylenchlorid. Ethylenchlorid. 1,2-Dichlorpropan, 1,2,3-Trichlorpropan oder Chlorbenzol umsetzt und beim Arbeiten in Chlorbenzol anschließend nach Zugabe eines Formamids Phosgen einleitet oder Thionylchlorid zutropft.

## Claims

1. Process for the preparation of substituted 2-chloropyridine derivatives of the formula (I) 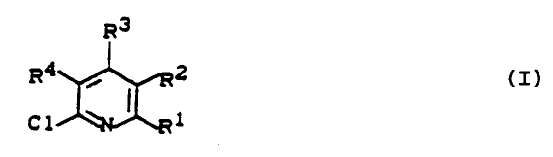 in which
R¹ represents hydrogen, fluorine, chlorine, cyano, carbalkoxy (C₁-C₄) or 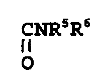 in which R⁵ and R⁶ are identical or different and represent alkyl (C₁-C₄),
R² represents hydrogen, fluorine, chlorine, alkyl(C₁-C₄),halogenoalkyl(C₁-C₄), cyanoalkyl(C₁-C₄), alkoxy(C₁-C₄)alkyl(C₁-C₄), dialkylamino(C₁-C₄)alkyl (C₁-C₄), acyl(C₁-C₄)-acetal, carbalkoxy(C₁-C₄) or 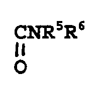 in which R⁵ and R⁶ are identical or different and represent alkyl (C₁-C₄)
or in which R¹ and R² together represent the divalent group -CH=CH-CH=CH-,
R³ represents hydrogen, chlorine, carbalkoxy(C₁-C₄) or 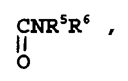 in which R⁵ and R⁶ are identical or different and represent alkyl (C₁-C₄),
R⁴ represents hydrogen, fluorine, chlorine or cyano, or in which R³ and R⁴ together represent the divalent group -CH=CH-CH=CH-,
characterized in that pyridine-l-oxides of the formula (II) 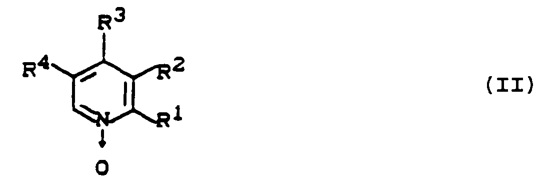 in which
R¹, R², R³ and R⁴ have the abovementioned meaning, are reacted with an aromatic carbonyl chloride in the presence of an inert organic solvent and in the presence of an acid acceptor at temperatures between -20°C and 200°C, then optionally reacted with phosgene or thionyl chloride at temperatures between 70°C and 160°C, if desired in the presence of an activator, and the product obtained is optionally further substituted.

2. Process according to Claim 1, characterized in that benzoyl chloride, monochlorobenzoyl chloride, dichlorobenzoyl chloride., monomethylbenzoyl chlorides, monochloromethylbenzoyl chlorides, dimethylbenzoyl chlorides, monomethoxybenzoyl chlorides, mononitrobenzoyl chlorides, phthaloyl chlorides, dichlorophthalide, monochlorophthaloyl chlorides, dichlorophthaloyl chlorides, mononitrophthaloyl chlorides and naphthalenecarbonyl chlorides are employed as aromatic carbonyl chlorides in the reaction.

3. Process according to Claims 1 and 2, characterized in that a basic organic nitrogen compound from the series comprising trialkylamines, dialkyl-cycloalkylamines and dialkylaralkylamines is employed as acid acceptor.

4. Process according to Claims 1 to 3, characterized in that triethylamine is employed as acid acceptor.

5. Process according to Claims 1 to 4, characterized in that a compound from the series comprising the formamides is employed as activator.

6. Process according to Claims 1 to 5, characterized in that methylene chloride, ethylene chloride, 1,1,2-trichloroethane, 1,2-dichloropropane, 1,2,3-trichloropropane, chloroform or chlorobenzene are employed as inert organic solvents.

7. Process according to Claims 1 to 6, characterized in that the reaction with the aromatic acid chloride is carried out at temperatures between 0 and 160°C and, if desired, subsequently with phosgene or thionyl chloride at temperatures between 70 and 160°C.

8. Process according to Claims 1 to 7, characterized in that 1 to 10 moles of an aromatic carbonyl chloride are added dropwise to a solution of 1 mole of the substituted pyridine-1-oxide of the formula (II) and of 1 to 10 moles of an amine, if desired a formamide is subsequently added and 2 to 5 moles of phosgene are introduced or thionyl chloride is added dropwise and the reaction mixture is stirred for several hours.

9. Process according to Claims 1 to 8, characterized in that the reaction mixture formed in the reaction is subjected to steam distillation.

10. Process according to Claims 1 to 9, characterized in that, in order to prepare 2-chloro-5-methylpyridine, 3-methyl-pyridine-1-oxide is reacted with a benzoyl chloride or phthaloyl chloride in methylene chloride, ethylene chloride, 1,2-dichloropropane, 1,2,3-trichloropropane or chlorobenzene with the addition of triethylamine as acid acceptor and, when working in chlorobenzene, phosgene is subsequently introduced or thionyl chloride is added dropwise after adding a formamide.

## Revendications

1. Procédé de production de dérivés substitués de 2-chloropyridine de formule (I) 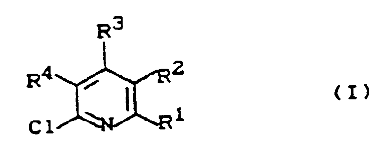 dans laquelle
R¹ représente l'hydrogène, le fluor, le chlore, un groupe cyano, carbalkoxy en C₁ à C₄ ou 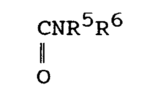 où R⁵ et R⁶ sont identiques ou différents et représentent un groupe alkyle en C₁ à C₄,
R² est de l'hydrogène, du fluor, du chlore, un groupe alkyle en C₁ à C₄, halogénalkyle en C₁ à C₄, cyanalkyle en C₁ à C₄, (alkoxy en C₁ à C₄)-(alkyle en C₁ à C₄), di(alkyle en C₁ à C₄)-amino-(alkyle en C₁ à C₄), (acyle en C₁ à C₄)-acétal, carbalkoxy en C₁ à C₄ ou 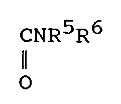 où R⁵ et R⁶ sont identiques ou différents et représentent un groupe alkyle en C₁ à C₄, ou bien R¹ et R² forment conjointement le groupe bivalent -CH=CH-CH=CH-,
R³ est de l'hydrogène, du chlore, un groupe carbalkoxy en C₁ à C₄ ou un groupe 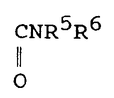 dans lequel R⁵ et R⁶ sont identiques ou différents et représentent un groupe alkyle en C₁ à C₄,
R⁴ est de l'hydrogène, du fluor, du chlore ou un groupe cyano,
ou bien R³ et R⁴ forment conjointement le groupe bivalent -CH=CH-CH=CH-,
caractérisé en ce qu'on fait réagir des 1-oxydes de pyridine de formule (II) 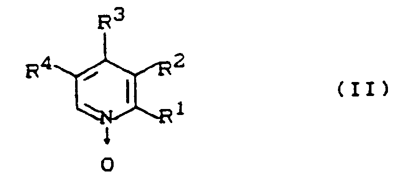 dans laquelle
R¹, R², R³ et R⁴ ont la définition indiquée ci-dessus, avec un chlorure d'acide carboxylique aromatique en présence d'un solvant organique inerte et en présence d'un accepteur d'acide, à des températures comprises entre -20°C et 200°C, après quoi, le cas échéant, on fait réagir le produit à des températures comprises entre 70°C et 160°C avec du phosgène ou du chlorure de thionyle, éventuellement en présence d'un activateur et on sépare ensuite le cas échéant le produit obtenu.

2. Procédé suivant la revendication 1, caractérisé en ce qu'on utilise comme chlorures d'acides carboxyliques aromatiques dans la réaction, le chlorure de benzoyle, les chlorures de monochlorobenzoyle, les chlorures de dichlorobenzoyle, les chlorures de monométhylbenzoyle, les chlorures de monochlorométhylbenzoyle, les chlorures de diméthylbenzoyle, les chlorures de monométhoxybenzoyle, les chlorures de mononitrobenzoyle, les chlorures de phtaloyle, le dichlorophtalide, les chlorures de monochlorophtaloyle, les chlorures de dichlorophtaloyle, les chlorures de mono-nitrophtaloyle, les chlorures d'acide naphtalènecarboxylique.

3. Procédé suivant les revendications 1 et 2, caractérisé en ce qu'on utilise comme accepteur d'acide un composé azoté organique basique de la série des trialkylamines, des dialkylcycloalkylamines et des dialkylaralkylamines.

4. Procédé suivant les revendications 1 à 3, caractérisé en ce qu'on utilise la triéthylamine comme accepteur d'acide.

5. Procédé suivant les revendications 1 à 4, caractérisé en ce qu'on utilise comme activateur un composé de la série des formamides.

6. Procédé suivant les revendications 1 à 5, caractérisé en ce qu'on utilise comme solvants organiques inertes le chlorure de méthylène, le chlorure d'éthylène, le 1,1,2-trichloréthane, le 1,2-dichloropropane, le 1,2,3-trichloropropane, le chloroforme ou le chlorobenzène.

7. Procédé suivant les revendications 1 à 6, caractérisé en ce qu'on conduit la réaction avec le chlorure d'acide aromatique à des températures comprises entre 0 et 160°C puis, le cas échéant, avec le phosgène ou le chlorure de thionyle à des températures comprises entre 70 et 160°C.

8. Procédé suivant les revendications 1 à 7, caractérisé en ce qu'on ajoute goutte à goutte à une solution d'une mole du 1-oxyde de pyridine substitué de formule (II) et de 1 à 10 moles d'une amine, 1 à 10 moles d'un chlorure d'acide carboxylique aromatique, on ajoute ensuite le cas échéant un formamide et on fait passer 2 à 5 moles de phosgène ou on ajoute goutte à goutte 2 à 5 moles de chlorure de thionyle, et on agite le mélange réactionnel pendant plusieurs heures.

9. Procédé suivant les revendications 1 à 8, caractérisé en ce qu'on soumet à une distillation à la vapeur d'eau le mélange réactionnel obtenu dans la réaction.

10. Procédé suivant les revendications 1 à 9, caractérisé en ce qu'on fait réagir, pour l'obtention de 2-chloro-5-méthylpyridine, le 1-oxyde de 3-méthylpyridine avec un chlorure de benzoyle ou un chlorure de phtaloyle avec addition de triéthylamine comme accepteur d'acide dans du chlorure de méthylène, du chlorure d'éthylène, du 1,2-dichloropropane, du 1,2,3-trichloropropane ou du chlorobenzène, et lorsqu'on opère dans du chlorobenzène, on fait ensuite passer du phosgène ou on ajoute goutte à goutte du chlorure de thionyle après l'addition d'un formamide.
